# EUROPEAN PATENT SPECIFICATION

(11) **EP 1 898 878 B1**
(45) Date of publication and mention of the grant of the patent: **08.01.2020**
(21) Application number: 06773731.2
(22) Date of filing: 21.06.2006
(51) Int. Cl.: A61K 9/00, A61K 9/48, A61K 9/20, A61F 13/00

(54) **DRUG/POLYMER COMPOSITE MATERIALS AND METHODS OF MAKING THE SAME**
ARZNEI/POLYMER-VERBUNDSTOFFE UND HERSTELLUNGSVERFAHREN DAFÜR
MATIERES COMPOSITES MEDICAMENT/POLYMERE ET PROCEDES DE FABRICATION DE CELLES-CI

(30) Priority: 22.06.2005 US 158724
(43) Date of publication of application: 19.03.2008
(73) Proprietor: Micell Technologies, Inc., Raleigh, NC 27617 (US)
(72) Inventor: MCCLAIN, Jim, Raleigh, NC 27613 (US); DEYOUNG, James, Dallas, TX 75214 (US)
(74) Representative: Wichmann, Hendrik
(86) International application number: PCT/US2006/024221
(87) International publication number: WO 2007/002238

(56) References cited:
- WO-A1-02/090085
- WO-A1-2004/098574
- WO-A1-2005/042623
- WO-A2-01/54662
- US-A- 3 087 860
- US-B1- 6 190 699

## Description

### FIELD OF THE INVENTION

The present invention concerns methods of making drug/polymer composite materials .

### BACKGROUND OF THE INVENTION

Drug/polymer composite materials are traditionally formed **either** by solvent-based processing where a solvent or combination of solvents is used to facilitate intimate mixing of the drug with polymer(s) by a combination of reducing the polymer viscosity and by dispersing/dissolving the drug into a fluid-like phase or by processing a mixture of drug(s) - polymer(s) at an elevated temperature sufficient to cause flow of the polymer into a desired shape. The solvents commonly utilized include all common organic solvents, halogenated solvents and aqueous solvent compositions. However, Solvent-based processing can adversely affect the drug by reacting, bonding or binding with the chemical functionality of many drugs. In addition, removal of solvent and solvent residues from the composite material is problematic and requires extensive processing with heat, vacuum, etc. Further, these processes can be process/cost intensive, lack precise material control and can adversely affect the drug. For example: *(i)* Trace solvent residues are unavoidable and are often toxic or can negatively interact with the drug or polymer molecules altering the therapeutic effect. *(ii)* Solvent-based processing can also adversely affect the primary structure of the drug in the polymer matrix. For example, making very difficult the production of small particles/domains of drug in the polymer matrix. *(iii)* Solvent-based processing can also adversely affect the secondary structure of sophisticated therapeutics such as proteins, enzymes, hormones, which changes the drug's efficacy and may denature the drug compound rendering it useless or toxic or change its effective shelf-life. *(iii)* Solvent-based processing can also adversely affect the polymorph of the drug; changing crystalline structure or providing amorphous materials that have different bioavailability profiles and adversely affecting shelf-life.

An alternative traditional process uses elevated temperatures to provide a lower viscosity polymer(s) for mixing with the drug. Again, however, high temperature processing can adversely affect many thermally sensitive drugs, rendering them ineffective or toxic, and elevated temperature processing is often used in conjunction with solvent-based methods (one still has to dissolve/disperse the drug molecule(s)), resulting in combined challenges of high temperature and solvents.

Densified gases, liquid and supercritical fluids have been described in the art as processing media for the incorporation of active materials including drugs into polymeric matrices. U.S. patent 5,340,614 (Perman) describes impregnating materials into polymeric matrices by using a carrier liquid that carries the active ingredient(s) where the carrier fluid is substantially insoluble in the supercritical fluid as is the active ingredient(s). A polymeric material is added to a pressure vessel after which the carrier liquid containing the active material(s) is(are) added, and then the system is exposed to supercritical carbon dioxide. After removal of the supercritical fluid and the carrier fluid, the polymer is found to have absorbed a portion of the active and presumably the carrier fluid.

U.S. Patent 6,190,699 (Luzzi) describes compositions of protein and peptide infused polymer particles and methods for production using densified solvents including supercritical fluids. Luzzi claims that the proteins and peptides are partially adsorbed into (infused) the polymer particles. Since proteins and peptides are not soluble in supercritical carbon dioxide, it can be reasonable assumed that dense carbon dioxide is not a suitable densified solvent to practice this art as sorption would be disfavored due to a lack of solubility of the protein in the densified solvent. Additionally, Luzzi discloses methods for making particles and does not address shaped or formed articles or semi-porous or porous articles.

What is needed in the art is a method that allows for the formation of polymer-drug composites that does not require the use of a carrier liquid or emulsions to make soluble or make mobile the drug for addition to the polymer. What is needed in the art is a method that allows for production of a polymer-drug composite that does not physically or chemically change the state of the drug during processing (solid to liquid). What is needed in the art is a method that allows for the creation of formed articles of a desired and controllable geometry. What is needed in the art is a method that allows for a low temperature forming of a semi-porous or porous solid article that does not physically or chemically change the state of the drug during processing.

Accordingly, there is a need for new approaches to the production of drug/polymer composite materials, and for new materials produced by such methods.

### SUMMARY OF THE INVENTION

Disclosed is a composite comprising at least one polymer; at least one pharmaceutical agent in a therapeutically desirable morphology; and optionally at least one excipient; wherein the pharmaceutical agent is sequestered in interstices formed by fusing particles of the polymer. The pharmaceutical agent is a drug or a biological agent having a secondary, tertiary and/or quaternary structure required for biological activity, wherein the structure is substantially retained when the particles of the biological agent are sequestered within interstices formed by the fused polymer particles. The pharmaceutical agent can be a peptide, protein, enzyme, nucleic acid, antibody, therapeutic vaccine, antisense nucleic acid, antimicrobial, vitamin, hormone, steroid, lipid, polysaccharide or carbohydrate. The pharmaceutical agent may be a therapeutic protein, such as erythropoietin, interferon, insulin, blood factor, colony stimulating factor, growth hormone, interleukin and growth factor. The pharmaceutical agent can be a drug in substantially crystalline form.

The composite may provide a rate of elution that remains within a 15% variation range from a selected rate of elution between day 2 and day 30 after the composite is implanted in a subject under physiological conditions.

The composite may provide an elution profile wherein about 10 % to about 50% of the pharmaceutical agent is eluted at week 1 after the composite is implanted in a subject under physiological conditions, about 25% to about 75% of the pharmaceutical agent is eluted at week 2 and about 50% to about 75% of the pharmaceutical agent is eluted at week 4. .

The polymer and the pharmaceutical agent may form two substantially separate phases.

The invention provides a method as defined in the claims.

In one embodiment, the method, further comprises forming a shaped article from said composite. In yet another embodiment, the shaped article is formed by placing the mixture of step (a) in a mold prior to performing said plasticizing of step (b).

In one embodiment, the shaped article is a biomedical implant., for example, a drug depot or a stent.

Further disclosed is a drug/polymer composite material (in some embodiments a "medicament" herein), which may be produced by a process as described above.

Also disclosed is a shaped article (in some embodiments also referred to as a "medicament" herein) comprising, consisting of or consisting essentially of a drug/polymer composite material as described above.

The drug/polymer composite materials can be used for treating a subject with a drug, comprising administering a drug/polymer composite material as described herein to said subject in an amount effective to treat said subject with said drug.

The foregoing and other objects and aspects of the present invention are explained in greater detail in the drawings herein and the specification set forth below.

### BRIEF DESCRIPTION OF THE DRAWINGS

FIGURE 1 shows supercritical CO₂ molding process and formation of an implant
FIGURE 2 shows supercritical CO₂ molding process "plasticization" leading to particle fusion according to the invention.
FIGURE 3 shows final implant, cross section SEM
FIGURE 4 shows In-vitro esculetin elution from PGLA implant
FIGURE 5 shows In-vitro esculetin mass elution from PGLA implant

### DETAILED DESCRIPTION OF THE PREFERRED EMBODIMENTS

The present invention is explained in greater detail below. This description is not intended to be a detailed catalog of all the different ways in which the invention may be implemented, or all the features that may be added to the instant invention. For example, features illustrated with respect to one embodiment may be incorporated into other embodiments.

In addition, numerous variations and additions to the various embodiments suggested herein will be apparent to those skilled in the art in light of the instant disclosure which do not depart from the instant invention. Hence, the following specification is intended to illustrate some particular embodiments of the invention, and not to exhaustively specify all permutations, combinations and variations thereof.

### A. Definitions.

Subjects that may be treated by the present invention include both human subjects for medical purposes and animal subjects for veterinary and drug screening and development purposes. Other suitable animal subjects are, in general, mammalian subjects such as primates, bovines, ovines, caprines, porcines, equines, felines, canines, rodents (*e.g*., rats and mice), *etc.* Human subjects are the most preferred. Human subjects include fetal, neonatal, infant, juvenile and adult subjects.

Shaped articles as used herein include, but are not limited to, pills, tablets, drug depots or drug delivery devices (*e.g*., subcutaneous implants), biomedical implants, etc.

"Biomedical implant" as used herein includes but is not limited to drug depots and drug delivery devices, stents (*e.g*., vascular stents), electrodes, catheters, leads, implantable pacemaker or cardioverter housings, joints, screws, rods, ophthalmic implants (including, but not limited to, intraocular lens implants, glaucoma implants or drainage implants, and punctal implants or plugs), etc. The implants may be of any suitable material, including but not limited to organic polymers (including stable or inert polymers and polymers), metals such as stainless steel and titanium, inorganic materials such as silicon, and composites thereof.

"Drug depot" or "drug delivery device" include those be configured for any route of administration, including those that may be implanted (luminal, venous, subcutaneous, muscular, ocular), inserted (oral, rectal, vaginal, ocular) or topically applied (transdermal, transmucual, sublingual).

"Treat" as used herein refers to any type of treatment or prevention that imparts a benefit to a subject afflicted with a disease or at risk of developing the disease, including improvement in the condition of the subject (e.g., in one or more symptoms), delay in the progression of the disease, delay the onset of symptoms or slow the progression of symptoms, etc. As such, the term "treatment" also includes prophylactic treatment of the subject to prevent the onset of symptoms. As used herein, "treatment" and "prevention" are not necessarily meant to imply cure or complete abolition of symptoms." to any type of treatment that imparts a benefit to a patient afflicted with a disease, including improvement in the condition of the patient (*e.g.*, in one or more symptoms), delay in the progression of the disease, etc.

"Pharmaceutical excipient" as used herein includes refers to any pharmaceutically acceptable material that is included in a drug composition to enhance the pharmaceutical (including manufacturing and shelf-stability) and/or pharmacological properties thereof. Pharmaceutical excipients include, but are not limited to, adjuvants, surfactants, stabilizers, morphology modifiers, porogens, diluents, carriers, solubilizers, antioxidants, lubricants (or glidants), binders, disintigrants, and mixtures thereof.

### Definitions

As used in the present specification, the following words and phrases are generally intended to have the meanings as set forth below, except to the extent that the context in which they are used indicates otherwise.

"Substrate" as used herein, refers to any surface upon which it is desirable to deposit a coating comprising a polymer and a pharmaceutical or biological agent, wherein the coating process does not substantially modify the morphology of the pharmaceutical agent or the activity of the biological agent. Biomedical implants are of particular interest for the present invention; however the present invention is not intended to be restricted to this class of substrates. Those of skill in the art will appreciate alternate substrates that could benefit from the coating process described herein, such as pharmaceutical tablet cores, as part of an assay apparatus or as components in a diagnostic kit (e.g. a test strip).

The implants may be formed from any suitable material, including but not limited to organic polymers (including stable or inert polymers and biodegradable polymers), metals, inorganic materials such as silicon, and composites thereof, including layered structures with a core of one material and one or more coatings of a different material.

In a preferred embodiment the biomedical implant is an expandable intraluminal vascular graft or stent (e.g., comprising a wire mesh tube) that can be expanded within a blood vessel by an angioplasty balloon associated with a catheter to dilate and expand the lumen of a blood vessel, such as described in US Patent No. 4,733,665 to Palmaz Shaz.

"Pharmaceutical agent" as used herein refers to any of a variety of drugs or pharmaceutical compounds that can be used as active agents to prevent or treat a disease (meaning any treatment of a disease in a mammal, including preventing the disease, i.e. causing the clinical symptoms of the disease not to develop; inhibiting the disease, i.e. arresting the development of clinical symptoms; and/or relieving the disease, i.e. causing the regression of clinical symptoms). It is possible that the pharmaceutical agents of the invention may also comprise two or more drugs or pharmaceutical compounds. Pharmaceutical agents, include but are not limited to antirestenotic agents, antidiabetics, analgesics, antiinflammatory agents, antirheumatics, antihypotensive agents, antihypertensive agents, psychoactive drugs, tranquillizers, antiemetics, muscle relaxants, glucocorticoids, agents for treating ulcerative colitis or Crohn's disease, antiallergics, antibiotics, antiepileptics, anticoagulants, antimycotics, antitussives, arteriosclerosis remedies, diuretics, proteins, peptides, enzymes, enzyme inhibitors, gout remedies, hormones and inhibitors thereof, cardiac glycosides, immunotherapeutic agents and cytokines, laxatives, lipid-lowering agents, migraine remedies, mineral products, otologicals, anti parkinson agents, thyroid therapeutic agents, spasmolytics, platelet aggregation inhibitors, vitamins, cytostatics and metastasis inhibitors, phytopharmaceuticals, chemotherapeutic agents and amino acids. Examples of suitable active ingredients are acarbose, antigens, beta-receptor blockers, non-steroidal antiinflammatory drugs {NSAIDs], cardiac glycosides, acetylsalicylic acid, virustatics, aclarubicin, acyclovir, cisplatin, actinomycin, alpha- and beta-sympatomimetics, (dmeprazole, allopurinol, alprostadil, prostaglandins, amantadine, ambroxol, amlodipine, methotrexate, S-aminosalicylic acid, amitriptyline, amoxicillin, anastrozole, atenolol, azathioprine, balsalazide, beclomethasone, betahistine, bezafibrate, bicalutamide, diazepam and diazepam derivatives, budesonide, bufexamac, buprenorphine, methadone, calcium salts, potassium salts, magnesium salts, candesartan, carbamazepine, captopril, cefalosporins, cetirizine, chenodeoxycholic acid, ursodeoxycholic acid, theophylline and theophylline derivatives, trypsins, cimetidine, clarithromycin, clavulanic acid, clindamycin, clobutinol, clonidine, cotrimoxazole, codeine, caffeine, vitamin D and derivatives of vitamin D, colestyramine, cromoglicic acid, coumarin and coumarin derivatives, cysteine, cytarabine, cyclophosphamide, ciclosporin, cyproterone, cytabarine, dapiprazole, desogestrel, desonide, dihydralazine, diltiazem, ergot alkaloids, dimenhydrinate, dimethyl sulphoxide, dimeticone, domperidone and domperidan derivatives, dopamine, doxazosin, doxorubizin, doxylamine, dapiprazole, benzodiazepines, diclofenac, glycoside antibiotics, desipramine, econazole, ACE inhibitors, enalapril, ephedrine, epinephrine, epoetin and epoetin derivatives, morphinans, calcium antagonists, irinotecan, modafinil, orlistat, peptide antibiotics, phenytoin, riluzoles, risedronate, sildenafil, topiramate, macrolide antibiotics, oestrogen and oestrogen derivatives, progestogen and progestogen derivatives, testosterone and testosterone derivatives, androgen and androgen derivatives, ethenzamide, etofenamate, etofibrate, fenofibrate, etofylline, etoposide, famciclovir, famotidine, felodipine, fenofibrate, fentanyl, fenticonazole, gyrase inhibitors, fluconazole, fludarabine, fluarizine, fluorouracil, fluoxetine, flurbiprofen, ibuprofen, flutamide, fluvastatin, follitropin, formoterol, fosfomicin, furosemide, fusidic acid, gallopamil, ganciclovir, gemfibrozil, gentamicin, ginkgo, Saint John's wort, glibenclamide, urea derivatives as oral antidiabetics, glucagon, glucosamine and glucosamine derivatives, glutathione, glycerol and glycerol derivatives, hypothalamus hormones, goserelin, gyrase inhibitors, guanethidine, halofantrine, haloperidol, heparin and heparin derivatives, hyaluronic acid, hydralazine, hydrochlorothiazide and hydrochlorothiazide derivatives, salicylates, hydroxyzine, idarubicin, ifosfamide, imipramine, indometacin, indoramine, insulin, interferons, iodine and iodine derivatives, isoconazole, isoprenaline, glucitol and glucitol derivatives, itraconazole, ketoconazole, ketoprofen, ketotifen, lacidipine, lansoprazole, levodopa, levomethadone, thyroid hormones, lipoic acid and lipoic acid derivatives, lisinopril, lisuride, lofepramine, lomustine, loperamide, loratadine, maprotiline, mebendazole, mebeverine, meclozine, mefenamic acid, mefloquine, meloxicam, mepindolol, meprobamate, meropenem, mesalazine, mesuximide, metamizole, metformin, methotrexate, methylphenidate, methylprednisolone, metixene, metoclopramide, metoprolol, metronidazole, mianserin, miconazole, minocycline, minoxidil, misoprostol, mitomycin, mizolastine, moexipril, morphine and morphine derivatives, evening primrose, nalbuphine, naloxone, tilidine, naproxen, narcotine, natamycin, neostigmine, nicergoline, nicethamide, nifedipine, niflumic acid, nimodipine, nimorazole, nimustine, nisoldipine, adrenaline and adrenaline derivatives, norfloxacin, novamine sulfone, noscapine, nystatin, ofloxacin, olanzapine, olsalazine, omeprazole, omoconazole, ondansetron, oxaceprol, oxacillin, oxiconazole, oxymetazoline, pantoprazole, paracetamol, paroxetine, penciclovir, oral penicillins, pentazocine, pentifylline, pentoxifylline, perphenazine, pethidine, plant extracts, phenazone, pheniramine, barbituric acid derivatives, phenylbutazone, phenytoin, pimozide, pindolol, piperazine, piracetam, pirenzepine, piribedil, piroxicam, pramipexole, pravastatin, prazosin, procaine, promazine, propiverine, propranolol, propyphenazone, prostaglandins, protionamide, proxyphylline, quetiapine, quinapril, quinaprilat, ramipril, ranitidine, reproterol, reserpine, ribavirin, rifampicin, risperidone, ritonavir, ropinirole, roxatidine, roxithromycin, ruscogenin, rutoside and rutoside derivatives, sabadilla, salbutamol, salmeterol, scopolamine, selegiline, sertaconazole, sertindole, sertralion, silicates, sildenafil, simvastatin, sitosterol, sotalol, spaglumic acid, sparfloxacin, spectinomycin, spiramycin, spirapril, spironolactone, stavudine, streptomycin, sucralfate, sufentanil, sulbactam, sulphonamides, sulfasalazine, sulpiride, sultamicillin, sultiam, sumatriptan, suxamethonium chloride, tacrine, tacrolimus, taliolol, tamoxifen, taurolidine, tazarotene, temazepam, teniposide, tenoxicam, terazosin, terbinafine, terbutaline, terfenadine, terlipressin, tertatolol, tetracyclins, teryzoline, theobromine, theophylline, butizine, thiamazole, phenothiazines, thiotepa, tiagabine, tiapride, propionic acid derivatives, ticlopidine, timolol, tinidazole, tioconazole, tioguanine, tioxolone, tiropramide, tizanidine, tolazoline, tolbutamide, tolcapone, tolnaftate, tolperisone, topotecan, torasemide, antioestrogens, tramadol, tramazoline, trandolapril, tranylcypromine, trapidil, trazodone, triamcinolone and triamcinolone derivatives, triamterene, trifluperidol, trifluridine, trimethoprim, trimipramine, tripelennamine, triprolidine, trifosfamide, tromantadine, trometamol, tropalpin, troxerutine, tulobuterol, tyramine, tyrothricin, urapidil, ursodeoxycholic acid, chenodeoxycholic acid, valaciclovir, valproic acid, vancomycin, vecuronium chloride, Viagra, venlafaxine, verapamil, vidarabine, vigabatrin, viloazine, vinblastine, vincamine, vincristine, vindesine, vinorelbine, vinpocetine, viquidil, warfarin, xantinol nicotinate, xipamide, zafirlukast, zalcitabine, zidovudine, zolmitriptan, zolpidem, zoplicone, zotipine and the like. See, e.g., US Patent No. 6,897,205; see also US Patent No. 6,838,528; US Patent No. 6,497,729.

The active ingredients may, if desired, also be used in the form of their pharmaceutically acceptable salts or derivatives (meaning salts which retain the biological effectiveness and properties of the compounds of this invention and which are not biologically or otherwise undesirable), and in the case of chiral active ingredients it is possible to employ both optically active isomers and racemates or mixtures of diastereoisomers.

"Active biological agent" as used herein refers to a substance, originally produced by living organisms, that can be used to prevent or treat a disease (meaning any treatment of a disease in a mammal, including preventing the disease, i.e. causing the clinical symptoms of the disease not to develop; inhibiting the disease, i.e. arresting the development of clinical symptoms; and/or relieving the disease, i.e. causing the regression of clinical symptoms). It is possible that the active biological agents of the invention may also comprise two or more active biological agents or an active biological agent combined with a pharmaceutical agent, a stabilizing agent or chemical or biological entity. Although the active biological agent may have been originally produced by living organisms, those of the present invention may also have been synthetically prepared, or by methods combining biological isolation and synthetic modification. By way of a non-limiting example, a nucleic acid could be isolated form from a biological source, or prepared by traditional techniques, known to those skilled in the art of nucleic acid synthesis. Furthermore, the nucleic acid may be further modified to contain non-naturally occurring moieties. Non-limiting examples of active biological agents include peptides, proteins, enzymes, glycoproteins, nucleic acids (including deoxyribonucleotide or ribonucleotide polymers in either single or double stranded form, and unless otherwise limited, encompasses known analogues of natural nucleotides that hybridize to nucleic acids in a manner similar to naturally occurring nucleotides), antisense nucleic acids, fatty acids, antimicrobials, vitamins, hormones, steroids, lipids, polysaccharides, carbohydrates and the like. They further include, but are not limited to, antirestenotic agents, antidiabetics, analgesics, antiinflammatory agents, antirheumatics, antihypotensive agents, antihypertensive agents, psychoactive drugs, tranquillizers, antiemetics, muscle relaxants, glucocorticoids, agents for treating ulcerative colitis or Crohn's disease, antiallergics, antibiotics, antiepileptics, anticoagulants, antimycotics, antitussives, arteriosclerosis remedies, diuretics, proteins, peptides, enzymes, enzyme inhibitors, gout remedies, hormones and inhibitors thereof, cardiac glycosides, immunotherapeutic agents and cytokines, laxatives, lipid-lowering agents, migraine remedies, mineral products, otologicals, anti parkinson agents, thyroid therapeutic agents, spasmolytics, platelet aggregation inhibitors, vitamins, cytostatics and metastasis inhibitors, phytopharmaceuticals and chemotherapeutic agents. Preferably, the active biological agent is a peptide, protein or enzyme, including derivatives and analogs of natural peptides, proteins and enzymes.

"Activity" as used herein refers to the ability of a pharmaceutical or active biological agent to prevent or treat a disease (meaning any treatment of a disease in a mammal, including preventing the disease, i.e. causing the clinical symptoms of the disease not to develop; inhibiting the disease, i.e. arresting the development of clinical symptoms; and/or relieving the disease, i.e. causing the regression of clinical symptoms). Thus the activity of a pharmaceutical or active biological agent should be of therapeutic or prophylactic value.

"Secondary, tertiary and quaternary structure " as used herein are defined as follows. The active biological agents of the present invention will typically possess some degree of secondary, tertiary and/or quaternary structure, upon which the activity of the agent depends. As an illustrative, non-limiting example, proteins possess secondary, tertiary and quaternary structure. Secondary structure refers to the spatial arrangement of amino acid residues that are near one another in the linear sequence. The α-helix and the *β*-strand are elements of secondary structure. Tertiary structure refers to the spatial arrangement of amino acid residues that are far apart in the linear sequence and to the pattern of disulfide bonds. Proteins containing more than one polypeptide chain exhibit an additional level of structural organization. Each polypeptide chain in such a protein is called a subunit. Quaternary structure refers to the spatial arrangement of subunits and the nature of their contacts. For example hemoglobin consists of two *α* and two *β* chains. It is well known that protein function arises from its conformation or three dimensional arrangement of atoms (a stretched out polypeptide chain is devoid of activity). Thus one aspect of the present invention is to manipulate active biological agents, while being careful to maintain their conformation, so as not to lose their therapeutic activity.

"Polymer" as used herein, refers to a series of repeating monomeric units that have been cross-linked or polymerized. Any suitable polymer can be used to carry out the present invention. It is possible that the polymers of the invention may also comprise two, three, four or more different polymers. In some embodiments, of the invention only one polymer is used. In some preferred embodiments a combination of two polymers are used. Combinations of polymers can be in varying ratios, to provide the polymer part of the composite materials of the invention including plasticized polymers that form a matrix with interstices wherein the drugs are sequestered. In addition to their role in forming the matrices of the invention, the polymers can be used as coating for composite materials of the invention. Selection of appropriate polymers can be employed in forming composite materials with differing properties. Those of skill in the art of polymer chemistry will be familiar with the different properties of polymeric compounds. "Polymer" as used herein refers to organic polymers, and includes copolymers of a named polymer with other constituents. In some embodiments, such as in the preparation of drug depots or drug delivery devices, the polymer is preferably an absorbable and/or resorbable polymer. In other embodiments the polymer is preferably non-resorbable and biocompatible. Examples of ploymers that may be used in the present invention include, but are not limited to polycarboxylic acids, cellulosic polymers, proteins, polypeptides, polyvinylpyrrolidone, maleic anhydride polymers, polyamides, polyvinyl alcohols, polyethylene oxides, glycosaminoglycans, polysaccharides, polyesters, polyurethanes, polystyrenes, copolymers, silicones, polyorthoesters, polyanhydrides, copolymers of vinyl monomers, polycarbonates, polyethylenes, polypropylenes, polylactic acids, polyglycolic acids, polycaprolactones, polyhydroxybutyrate valerates, polyacrylamides, polyethers, polyurethane dispersions, polyacrylates, acrylic latex dispersions, polyacrylic acid, mixtures and copolymers thereof. The polymers of the present invention may be natural or synthetic in origin, including gelatin, chitosan, dextrin, cyclodextrin, Poly(urethanes), Poly(siloxanes) or silicones, Poly(acrylates) such as poly(methyl methacrylate), poly(butyl methacrylate), and Poly(2-hydroxy ethyl methacrylate), Poly(vinyl alcohol) Poly(olefins) such as poly(ethylene), poly(isoprene), halogenated polymers such as Poly(tetrafluoroethylene) - and derivatives and copolymers such as those commonly sold as Teflon® products, Poly(vinylidine fluoride), Poly(vinyl acetate), Poly(vinyl pyrrolidone),. Poly(acrylic acid), Polyacrylamide, Poly(ethylene-co-vinyl acetate), Poly(ethylene glycol), Poly(propylene glycol), Poly(methacrylic acid); etc. Suitable polymers also include absorbable and/or resorbable polymers including the following, combinations, copolymers and derivatives of the following: Polylactides (PLA), Polyglycolides (PGA), Poly(lactide-co-glycolides) (PLGA), Polyanhydrides, Polyorthoesters, Poly(N-(2-hydroxypropyl) methacrylamide), Poly(l-aspartamide), etc.

Any suitable polymer can preferably be used to carry out the present invention, including but not limited to: natural and synthetic polymers, gelatin, chitosan, dextrin, cyclodextrin, Poly(urethanes), Poly(siloxanes) or silicones , Poly(acrylates) such as poly(methyl methacrylate), poly(butyl methacrylate), and Poly(2-hydroxy ethyl methacrylate), Poly(vinyl alcohol) Poly(olefinds) such as poly(ethylene), poly(isoprene), halogenated polymers such as Poly(tetrafluoroethylene) - and derivatives and copolymers such as those commonly sold as Teflon® products, Poly(vinylidine fluoride), Poly(vinyl acetate), Poly(vinyl pyrrolidone),. Poly(acrylic acid), Polyacrylamide, Poly(ethylene-co-vinyl acetate), Poly(ethylene glycol), Poly(propylene glycol), Poly(methacrylic acid); *etc.* Suitable polymers also include absorbable and/or resorbable polymers including the following, combinations, copolymers and derivatives of the following: Polylactides (PLA), Polyglycolides (PGA), Poly(lactide-co-glycolides) (PLGA), Polyanhydrides, Polyorthoesters, Poly(*N*-(2-hydroxypropyl) methacrylamide), Poly(l-aspartamide), *etc.*

"Therapeutically desirable morphology" as used herein refers to the gross form and structure of the pharmaceutical agent, once deposited on the substrate, so as to provide for optimal conditions of ex vivo storage, in vivo preservation and/or in vivo release. Such optimal conditions may include, but are not limited to increased shelf life, increased in vivo stability, good biocompatibility, good bioavailability or modified release rates. Typically, for the present invention, the desired morphology of a pharmaceutical agent would be crystalline or semi-crystalline, although this may vary widely depending on many factors including, but not limited to, the nature of the pharmaceutical agent, the disease to be treated/prevented, the intended storage conditions for the substrate prior to use or the location within the body of any biomedical implant. Preferably at least 10%, 20%, 30%, 40%, 50%, 60%, 70%, 80%, 90% or 100% of the pharmaceutical agent is in crystalline or semi-crystalline form.

"Stabilizing agent" as used herein refers to any substance that maintains or enhances the stability of the biological agent. Ideally these stabilizing agents are classified as Generally Regarded As Safe (GRAS) materials by the US Food and Drug Administration (FDA). Examples of stabilizing agents include, but are not limited to carrier proteins, such as albumin, gelatin, metals or inorganic salts.

"Supercritical fluid", "near-critical fluid", "critical fluid", "densified fluid" or "densified gas" as used herein refers to a substance under pressure greater than ambient conditions, where it demonstrates a density greater than 0.4 g/cc but the mobility of a gas, i.e. a gas with liquid-like densities in which the pressure and temperature are above the critical point (the temperature and pressure at which the density of the liquid and vapor phases become identical). Examples of near critical fluids include fluids which are in gaseous state at standard temperature and pressure (STP) conditions and have a critical density above 0.2 g/cc. See, e.g., US Patent Nos. 6,860,123; 6,837,611; and 6,755,871. Examples of substances that demonstrate supercritical or near critical behavior suitable for the present invention include, but are not limited to carbon dioxide, ammonia, water, methanol, ethanol, ethane, propane, butane, pentane, dimethyl ether, xenon, sulfur hexafluoride, halogenated and partially halogenated materials such as chlorofluorocarbons, hydrochlorofluorocarbons, hydrofluorocarbons, perfluorocarbons (such as perfluoromethane and perfuoropropane, chloroform, trichloro-fluoromethane, dichloro-difluoromethane, dichloro-tetrafluoroethane) and mixtures thereof. Carbon dioxide is preferred. For densified carbon dioxide, conditions including a temperature between 0° C and 100° C and a pressure between 30 psig and 10,000 psig are preferred.

"Sintering" or "plasticizing" as used herein refers to the process by which the codeposited pharmaceutical agent or biological agent-polymer matrix, as described above, becomes fused by treatment of the polymer matrix or substrate with a compressed gas, compressed liquid, or densified fluid that is a non-solvent for both the polymer and the pharmaceutical agent and biological agents, but a plasticizing agent for the polymer. In one example, carbon dioxide is used to treat a matrix of polymer or a substrate that has been coated with a polymer and a drug, using dry powder and RESS electrostatic coating processes.

"Bulk properties" properties of a coating including a pharmaceutical or a biological agent that can be enhanced through the methods of the invention include for example: adhesion, smoothness, conformality, thickness, and compositional mixing.

"Rapid Expansion of Supercritical Solutions" or "RESS" as used herein involves the dissolution of a polymer into a compressed fluid, typically a densified fluid, followed by rapid expansion into a chamber at atmospheric pressure. The rapid expansion of the densified fluid solution through a small opening, with its accompanying decrease in density, reduces the dissolution capacity of the fluid and results in the nucleation and growth of polymer particles.

"Solution Enhanced Dispersion of Supercritical Solutions" or "SEDS" as used herein involves a spray process for the generation of polymer particles, which are formed when a compressed fluid (e.g. densified fluid, preferably densified CO2) is used as a diluent to a vehicle in which a polymer or the drug is dissolved, (one that can dissolve both the polymer or the drug and the densified gas). The mixing of the densified fluid diluent with the polymer-containing solution may be achieved by encounter of a first stream containing the polymer solution and a second stream containing the diluent densified fluid, for example, within one spray nozzle or by the use of multiple spray nozzles. The solvent in the polymer solution may be one compound or a mixture of two or more ingredients and may be or comprise an alcohol (including diols, triols, etc.), ether, amine, ketone, carbonate, or alkanes, or hydrocarbon (aliphatic or aromatic) or may be a mixture of compounds, such as mixtures of alkanes, or mixtures of one or more alkanes in combination with additional compounds such as one or more alcohols. (e.g., from 0 or 0.1 to 5% of a C1 to C15 alcohol, including diols, triols, etc.). See for example US Patent No. 6,669,785. The solvent may optionally contain a surfactant, as also described in (for example) US Patent No. 6,669,785.

In one embodiment of the SEDS process, a first stream of fluid comprising a polymer dissolved in a common solvent is co-sprayed with a second stream of densified fluid. Polymer particles are produced as the second stream acts as a diluent that weakens the solvent in the polymer solution of the first stream. The now combined streams of fluid, along with the polymer particles, flow out into a collection vessel which may or may not be at elevated pressure. In another embodiment of the SEDS process, a first stream of fluid comprising a drug dissolved in a common solvent is co-sprayed with a second stream of densified fluid. Drug particles are produced as the second stream acts as a diluent that weakens the solvent in the drug solution of the first stream. The now combined streams of fluid, along with the drug particles, flow out into a collection vessel which may or may not be at elevated pressure.

Control of particle size, particle size distribution, and morphology is achieved by tailoring the following process variables: temperature, pressure, solvent composition of the first stream, flow-rate of the first stream, flow-rate of the second stream, composition of the second stream (where soluble additives may be added to the densified gas), and conditions of the capture vessel. Typically the capture vessel contains a fluid phase that is at least five to ten times (5-10x) atmospheric pressure.

"Electrostatically charged" or "electrical potential" or "electrostatic capture" as used herein refers to the collection of the spray-produced particles upon a substrate that has a different electrostatic potential than the sprayed particles. Thus, the substrate is at an attractive electronic potential with respect to the particles exiting, which results in the capture of the particles upon the substrate. i.e. the substrate and particles are oppositely charged, and the particles transport through the fluid medium of the capture vessel onto the surface of the substrate is enhanced via electrostatic attraction. This may be achieved by charging the particles and grounding the substrate or conversely charging the substrate and grounding the particles, or by some other process, which would be easily envisaged by one of skill in the art of electrostatic capture.

"Open vessel" as used herein refers to a vessel open to the outside atmosphere, and thus at substantially the same temperature and pressure as the outside atmosphere.

"Closed vessel" as used herein refers to a vessel sealed from the outside atmosphere, and thus may be at significantly different temperatures and pressures to the outside atmosphere.

The drug or active ingredient may be in any physical form, such as crystalline (including semicrystalline) and amorphous.

### Solvents.

Solvents that may be used to carry out the present invention are, in some embodiments, gases (that is, compounds that are in the form of a gas at atmospheric pressure and 25 °C). Examples of such solvents include but are not limited to carbon dioxide, ammonia, water, methanol, ethanol, ethane, propane, butane, pentane, dimethyl ether, xenon, sulfur hexafluoride, halogenated and partially halogenated materials such as chlorofluorocarbons, hydrochlorofluorocarbons, hydrofluorocarbons, perfluorocarbons (such as perfluoromethane and perfuoropropane, chloroform, trichloro-fluoromethane, dichloro-difluoromethane, dichloro-tetrafluoroethane) and mixtures thereof. Carbon dioxide is preferred.

The solvent may be utilized per se or a cosolvent may be included therewith (e.g., in an amount of from 0.01 or 0.1 to 20 or 30 percent by weight or more). Examples of cosolvents include, but are not limited to, water and organic co-solvents. The organic co-solvent may be one compound or a mixture of two or more ingredients. The organic co-solvent may be or comprise an alcohol (including diols, triols, etc.), ether, amine, ketone, carbonate, or alkanes, or hydrocarbon (aliphatic or aromatic) The organic co-solvent may be a mixture of compounds, such as mixtures of alkanes as given above, or mixtures of one or more alkanes in combination with additional compounds such as one or more alcohols as described above. (e.g., from 0 or 0.1 to 5% of a C1 to C15 alcohol (including diols, triols, etc.)). *See, e.g.,* US Patent No. 6,669,785. The solvent may optionally contain a surfactant, as also described in (for example) US Patent No. 6,669,785.

The solvent is preferably provided in densified form. This densified form can be a gas at densities greater than 1.1 times the gas density at STP, a liquid (including near-supercritical fluids) or as a densified fluid, these three forms together sometimes being referred to as a "densified" fluid or "densified" gas. *See, e.g.,* US Patent Nos. 6,860,123; 6,837,611; and 6,755,871.

### Excipients.

Numerous pharmaceutical excipients that may be used to carry out the present invention are known. *See, e.g.,* US Patent Nos. 6,767,558; 6,720,003; 6,710,059; and 6,649,627. Comprehensive examples are included in the Handbook of Pharmaceutical Excipients, edited by Raymond Rowe, Paul Sheskey and Paul Weller (4th Ed. 2003). Among other things, the drug-polymer composition may contain pharmaceutical excipients materials for: 1) enhancing the stability of the drug, 2) modifying the ultimate morphology of the drug or polymer, or drug polymer composite 3) inserting a porogen into the composite for subsequent removal in or during dense fluid processing, 4) improving the solubility characteristics of the drug in-vitro and in-vivo. Ideally these excipients are classified as Generally Regarded As Safe (GRAS) materials by the US Food and Drug Administration (FDA).

In category '1' above the excipient serves to stabilize the drug material. A primary example is represented by the use of sugars and other carbohydrates to stabilize proteins and peptides in pharmaceutical formulations. In the current invention one particularly useful sugar derivative is Sucrose Octa Acetate (SOA) which can serve to stabilize proteins in solution or in the solid state during compounding of the drug with the polymer. The SOA may also serve to benefit the composite in downstream processing described below.

In category '2' above the excipient serves to modify the morphology of the drug or polymer or the composite during and after processing with a dense gas fluid. One highlighted advantage to using dense gas fluids for processing drug-polymer composites relates to the "plasticizing" effect of the fluid (such as densified carbon dioxide) on the polymer. The fluid essentially permeates the free-volume of the polymer micro-structure lowering the glass transition temperature of the amorphous polymer and enhancing particle fusion at temperature much lower than those needed for heat bonding or fusion. This enhanced flow allows for suitable cohesion or adhesion of the formulated drug-polymer composite creating a semi-rigid composite product. The inclusion of excipients such as SOA may also serve to further plasticize the polymer thus enhancing the particle fusion and the overall solid-state integrity of the final composite.

In category '3' above the excipient serves as a removable material (porogen) during the dense fluid processing step. For non-absorbable polymers it may be desirable to create increased surface area to affect drug removal in-vivo. By inclusion of the excipient material during the compounding step, a porous or semi-porous structure is created upon exposure to the dense fluid. In this case, the excipient is extracted from the formed composite leaving a micro- or nano-porous internal structure after completed dense fluid processing. One particular excipient of interest is SOA. Sucrose octaacetate is know to be soluble in dense carbon dioxide and in this case may serve as a stabilizer, a plasticizer, and a porogen. Other partially or fully acetylated sugars and carbohydrates may also be employed for these same purposes.

In category '4' above the excipient increases the solubility of the drug as measure in-vitro and as applied in-vivo by preventing drug aggregation/agglomeration and by increasing the hydration capacity of the drug particle in-situ. Many drugs have poor aqueous solubility and therefore limited efficacy based on there ability to reach sufficient levels in the blood. Aside from particle size control (smaller particle size equals better dissolution profiles) excipients are used to prevent particle agglomeration and to enhance dissolution characteristics by increasing hydration in and around the particle. Noteworthy examples useful in the current invention include dextrin and its derivatives, other carbohydrates and simple sugars, and partially or fully acetylated sugars such as SOA.

As outlined above the excipient may serve one or several of the purposes described.

Other useful excipients include surfactants. Ideally, these surfactants are classified as GRAS materials by the FDA. Suitable examples include but are not limited to sorbitan monooleate, Twean® trademarked surfactants, soy derived surfactants, and fatty acid derived GRAS surfactants, These surfactants may serve one or multiple roles as described above in this section.

As indicated above, SOA and other such hydrophobically derivatized carbohydrates (HDCs) can be utilized as the pharmaceutical excipient. HDCs are a wide variety of hydrophobically derivatized carbohydrates where at least one hydroxyl group is substituted with a hydrophobic moiety including, but not limited to, esters and ethers. Numerous examples of suitable HDCs and their syntheses are described in Developments in Food Carbohydrate, C. K. Lee, Applied Science Publishers, London (2d Ed. 1980) and PCT publication No. 96/03978. Other syntheses are described in, for example, Akoh et al. (1987) J. Food Sci. 52:1570; Khan et al. (1933) Tetra. Letts 34:7767; Khan (1984) Pure & Appl. Chem. 56:833-844; and Khan et al. (1990) Carb. Res, 198:275-283. Specific examples of HDCs include, but are not limited to, sorbitol hexaacetate (SHAC), alpha-glucose penta acetate (alpha-GPAC), beta-glucose pentaacetate (beta-GPAC), 1-O-Octyl-beta-D-glucose tetraacetate (OGTA), trehalose octaacetate (TOAC), trehalose octapropionate (TOP), trehalose octa-3,3,dimethylbutyrate (TO33DMB), trehalose diisobutyrate hexaacetate, trehalose octaisobutyrate, lactose octaacetate, sucrose octaacetate (SOAC), cellobiose octaacetate (COAC), raffinose undecaacetate (RUDA), sucrose octapropanoate, cellobiose octapropanoate, raffinose undecapropanoate, tetra-O-methyl trehalose, trehalose octapivalate, trehalose hexaacetate dipivalate and di-O-methyl-hexa-O-actyl sucrose and mixtures thereof, *See, e.g.,* US Patent No. 6,517,860.

### Methods of making and using.

The method of the invention may be carried out by first, combining the drug with the polymer and optionally an excipient(s) to form a mixture. This mixing step may be carried out by any suitable technique or in any suitable apparatus, such as in a blender, extruder, etc. Typically both the drug and the polymer are provided in solid particulate form, and hence the mixture so formed will also be in the form of a solid.

Typically the polymer and the drug particles range between 0.02 and 50 microns in size. In some embodiments the particle size is in a larger size range than the drug. In this case the polymer may range from 0.2 micron and 50 microns and the drug from 0.02 to 20 microns.

Next, the mixture is contacted under pressure with a supercritical gas solvent as described above to form the composite material. Without wishing to be bound to any particular theory of the invention, it is believed that the densified gas solvent is at a pressure sufficient to reduce the viscosity of the polymer material, trapping the fluid insoluble drug material in the polymer matrix as polymer particles fuse with adjacent polymer particles and hence form the drug/polymer composite article. As contrasted with other art utilizing dense fluid gases such as carbon dioxide at high pressures, many drugs, particularly protein-based drugs, are not soluble in the dense fluid and therefore are not efficiently infused into polymer matrices. In the current invention the drug, such as a protein-based therapeutic may remain largely unchanged as the polymer particle fuse around the drug particles. Depending upon the specific manner in which this step is carried out the drug/polymer composite can be in the form of discrete particles (which may for example be the same size but likely larger than the polymer particles previously provided) or may be in the form of a shaped article. Ideally, the composite mixture is used in conjunction with a mechanical article such as a mold or a template and the final composite article takes on the shape or general shape of that mold or template. So in working practice the mixture of the drug, polymer and excipients is added to a three-dimensional article, mechanically constrained such that the particles of both the drug and the polymer are immobilized. The densified fluid at the desired pressure and temperature is then allowed to permeate the three-dimensional article such to effect the fusion of the polymer particles without extraction or removal of either the drug or the polymer from the mechanical article. Finally the fluid is removed from the mechanical article by reducing the pressure to ambient levels and the final composite is then removed from the template as a semi-rigid solid composite. In general, this contacting step is carried out at a pressure between 300 and 15,000 psig and a temperature of between 20C and 175 °C. Most preferably the contacting step is carried out at between300 and 3000 psig at a temperature between 20C and 100 °C

The step of combining the mixture with the solvent can be carried out by any suitable technique or in any suitable apparatus, such as in an extruder (which may be the same or different from the extruder noted above), mold (*e.g.*, injection mold, blow mold, compression mold, etc.), reaction vessel, etc. A shaped article as described herein may, in some embodiments, be formed concurrently with this combining step, for example when the combining is carried out in a mold, or when the combining is carried out in an extruder and the composite formed therein then extruded through a die. In other embodiments, however, the shaped article will be formed in a subsequent step. Such subsequent forming may likewise be carried out by any suitable technique such as by spraying or dipping a pre-formed substrate with the composite material (*e.g*., to form a stent or biomedical implant). By use of a subsequent extruder or mold, etc.

The drug/composite material may comprise, consist of, or consist essentially of:
from 0.01 or 0.1 percent to 40, 50, or 60 percent by weight of drug (which may be a single compound or a combination of different active agents); and
from 40 or 50 percent to 99.9 or 99.99 percent by weight of polymer;
optionally, from 0.01 or 0.1 percent to 20 or 30 percent pharmaceutical excipient.

In some embodiments, the physical form of the drug in the composite is substantially the same as the physical form of the drug before the combining step (b). For example, a drug initially provided in crystalline form remains in crystalline form in the composite; a drug initially provided in amorphous form remains in amorphous form in the composite; etc.

In some embodiments, the composite is porous (this term including "semiporous"), with porous composites being made by inclusion of a porogen as a pharmaceutical excipient and subsequent removal of at least a portion thereof by an appropriate solvent (e.g., organic solvents; densified carbon dioxide solvent compositions as described herein) thereof after formation of the composite, in accordance with known techniques. In some embodiments the porogen is an SOA or other such hydrophobically derivatized carbohydrate as described above.

*Secondary coatings.* Drug/polymer composites prepared as described above may optionally be coated (*e.g.*, by spraying, dipping, or any suitable technique) with a second material to aid in the subsequent binding, forming, dispersion, structure or drug-elution profile of the drug/ polymer composite. This second material can be any of several different chemical functionalities and several different functions in the resulting drug/polymer composite material. For example, the second material can be a pharmaceutical excipient, providing a means to alter the pharmacological effect of the drug or providing a means to alter the release profile of the drug-delivery. In some embodiments the second material can be a CO₂-philic material. In this case an additional process step can be utilized where after compressive forming of the part, a second condition of densified fluid can be used to remove the CO₂-philic material, thereby forming pores in and rendering porosity to the formed part.

The present invention is explained in greater detail in the following non-limiting Examples.

### Example 1

### Preparation of a Drug Polymer Composite Article using Densified Fluid Processing

A cylindrical composite article consisting of 3 parts poly(butyl methacrylate), 2 parts recombinant Human Growth hormone (rHGh), and 1 part sucrose octaacetate is created in the following manner. Spherical emulsion prepared poly(butyl methacrylate) of an average size range of 3.0 microns is blended with lyophilized HGh with an average particle size of 1.0 microns using an ultrasonic mixer. Dry sucrose octaacetate powder in the appropriate ratio is then added under constant mixing. The resulting formulation is then added to a cylindrical hollow mold constructed from sintered metal creating a fluid permeable three-dimensional article with an average pore size of 0.2 microns. The cylinder is open on both ends. Prior to the addition of the drug-polymer composition to the mold, one end is closed off using a matching cap designed to lock in place at the end of the cylinder. Once added to the mold, the composition is then mechanically compressed using a metal plunger matching the approximate inner diameter of the cylinder minus 0.001 -inch to remove the majority of the free-volume. The other end of the cylinder is then closed using an end cap that locks in place constraining the composition in three dimensions. The mold containing the polymer drug composition is then placed in a sterile pressure vessel to which 99.99% pure carbon dioxide is added to a pressure of 4000 psig at a temperature of 80C. The article is maintained in the CO2 environment at this temperature for 20 minutes after which the vessel is vented to atmospheric conditions. The mold is then removed from the vessel and the end caps are removed. The drug-polymer composite is then removed from the mold using a metal plunger fed from the open top of the mold thus pushing the composite out the bottom as the cylinder is mechanically restrained. Upon inspection the sample is a semi-rigid solid article in the shape of the mold. Upon thorough analysis of the polymer drug composite using Scanning Electron Microscopy (SEM) and routine chemical analysis it is determined that the solid article consists of a porous network of fused polymer particles with protein residing largely between adjacent fused particles and in void spaces created by the partial extraction of the sucrose octaacetate. Upon detailed morphological and chemical examination of the composite it is determined that the porous structure is largely inter-connected and partially opened to the outer surface of the article and the ratio of polymer to drug to sucrose octaacetate was 3:2:0.2 indicating substantial removal of the sucrose derivative during fluid processing.

### Example 2

### Preparation of a Drug Polymer Composite Article using Densified Fluid Processing

A cylindrical composite article consisting of 4 parts poly(butyl methacrylate), 2 parts recombinant Human Growth hormone (rHGh), and 2 part sucrose octaacetate is created in the following manner. Spherical emulsion prepared poly(butyl methacrylate) of an average size range of 10.0 microns is blended with lyophilized HGh with an average particle size of 1.0 microns using an ultrasonic mixer. Dry sucrose octaacetate powder in the appropriate ratio is then added under constant mixing. The resulting formulation is then added to a cylindrical hollow mold constructed from sintered metal creating a fluid permeable three-dimensional article with an average pore size of 0.2 microns. The cylinder is open on both ends. Prior to the addition of the drug-polymer composition to the mold, one end is closed off using a matching cap designed to lock in place at the end of the cylinder. The mold containing the polymer-drug-excipient mixture is then added to a pressure vessel equipped with a mechanical device designed with a piston actuator to exert pressure on the open end of the mold. The sealed pressure vessel is then filled with densified CO2 to a pressure of 3000 psi at a temperature of 80C. After 5 minutes at static pressure and temperature, the piston is actuated to apply mechanical pressure through the open end of the mold compressing the composition with 25 lbs-(in²)⁻¹ of mechanical force. After 5 minutes of mechanical compression and exposure to CO2 at a pressure of 3000 psi (80C) the CO2 is vented from the chamber and the piston is removed from the open end of the cylindrical mold. The drug-polymer composite is then removed from the mold using a metal plunger fed from the open top of the mold thus pushing the composite out the bottom as the cylinder is mechanically restrained. Upon inspection the sample is a semi-rigid solid article in the shape of the mold.

### Example 3

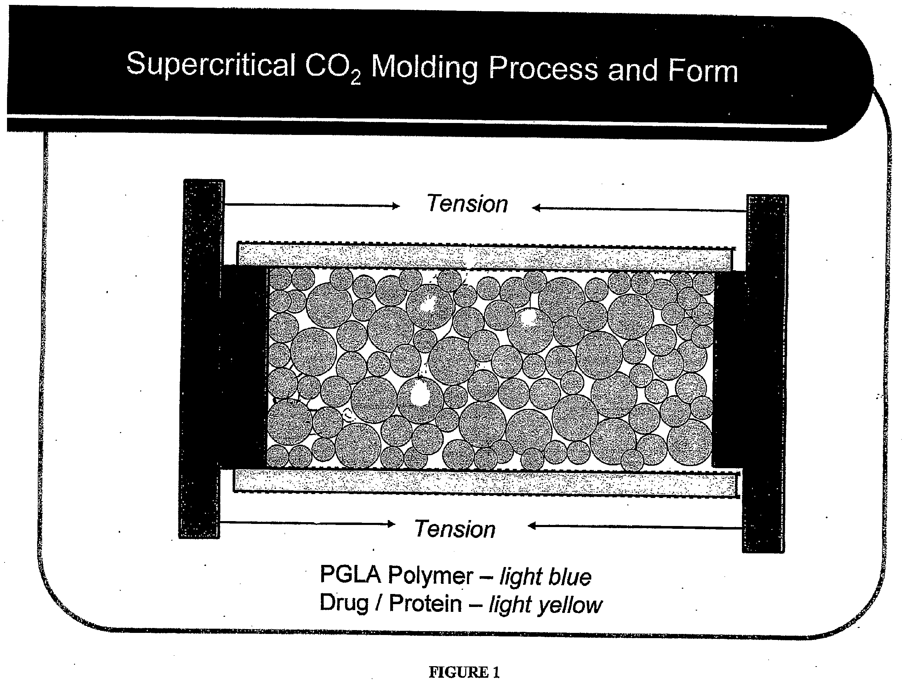

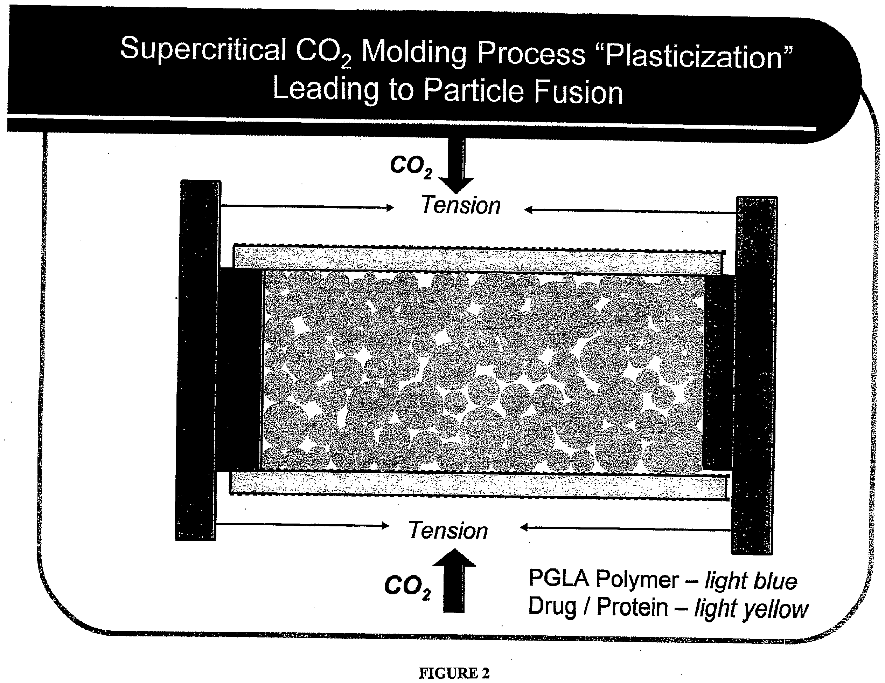

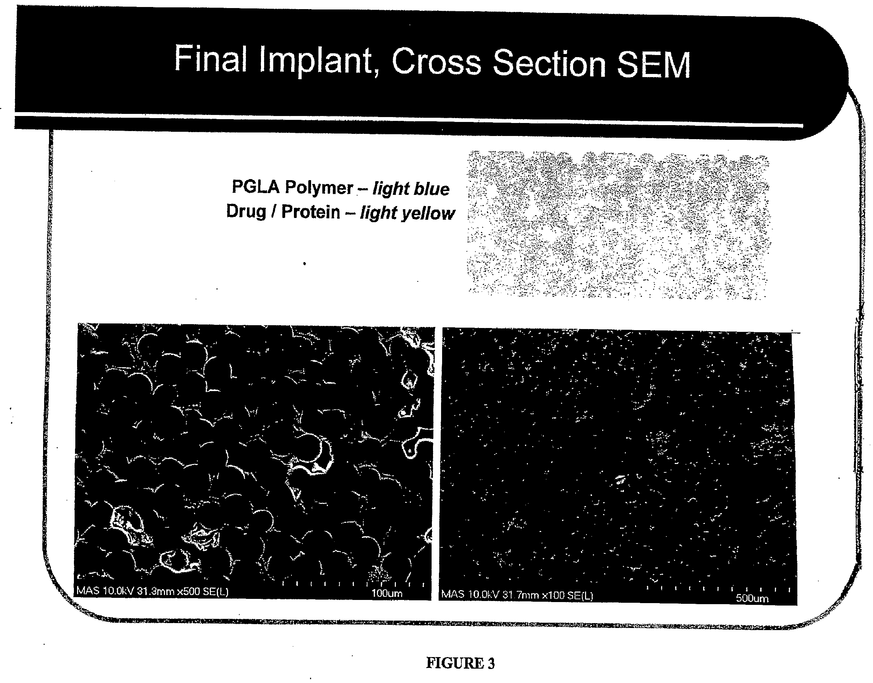

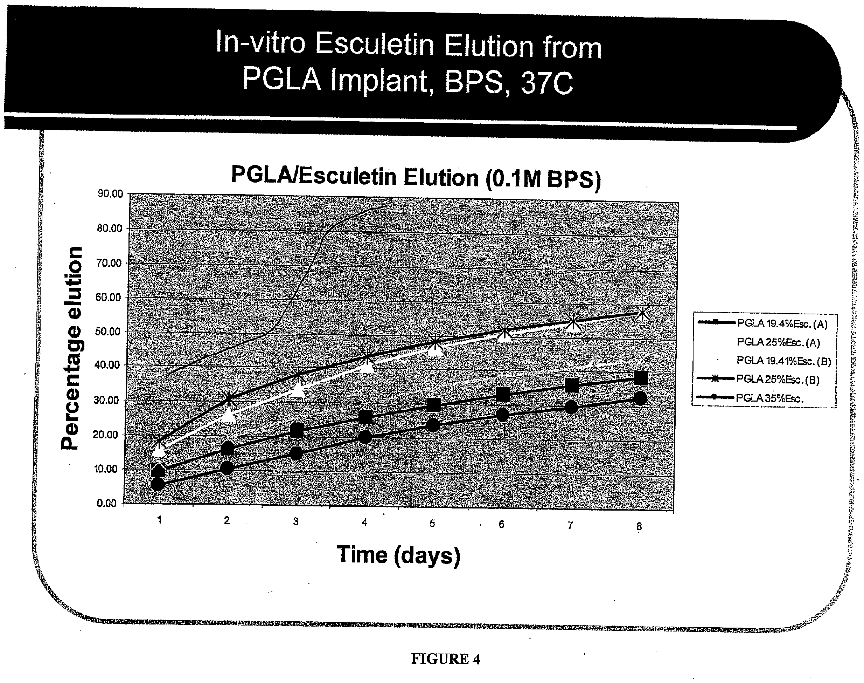

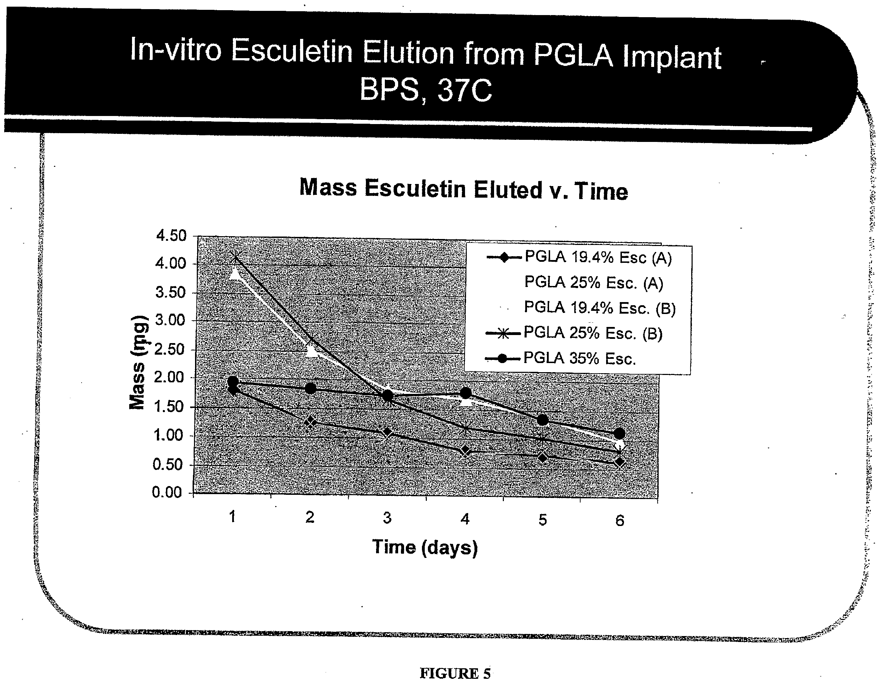

Figures 1-5 illustrate embodiments of the invention including SEM images of fused PGLA matrix formed according to the invention with and without drug present in the interstices formed by fusing the PGLA polymer. Elution profiles for an implant according to the invention formed with PGLA as the polymer and esculetin as the drug are also shown.

## Claims

1. A method of forming a drug/polymer composite material, comprising the steps of:
(a) mixing a solid particulate drug material with a solid particulate polymer material, and a pharmaceutical excipient, to form a particle mixture of polymer particles and interspersed drug particles; and then
(b) combining said particle mixture with a supercritical gas solvent at a pressure sufficient to reduce the viscosity of said polymer material, fuse said polymer particles to one another and capture said drug particles therebetween and form a drug/polymer composite material from said particulate mixture, wherein said excipient is a porogen, said method further comprising the step of contacting said composite material to a solvent to at least partially solubilize said porogen and form pores in said composite material.

2. The method of claim 1, wherein said combining step (b) is carried out in a mold so that a shaped article of said drug/polymer composite material is thereby produced.

3. The method of claim 2, wherein said shaped article is a stent, drug depot, or biomedical implant.

4. The method of claim 1, further comprising the step:
(c) forming a shaped article from said drug/polymer composite material.

5. The method of claim 4, wherein said combining step (b) is carried out in an extruder.

6. The method of claim 4, wherein said forming step (c) is carried out by coating a pre-formed substrate with said drug/polymer composite material.

7. The method of claim 4, wherein said shaped article is a stent, drug depot, or biomedical implant.

8. The method of claim 1, wherein said drug is a protein or peptide.

9. The method of claim 1, further comprising the step of coating said composite material with a secondary material.

## Patentansprüche

1. Verfahren zum Bilden eines Arzneimittel/Polymer-Verbundmaterials, umfassend die Schritte:
(a) Mischen eines festen partikelförmigen Arzneimittelmaterials mit einem festen partikelförmigen Polymermaterial und einem pharmazeutischen Hilfsstoff, um eine Partikelmischung aus Polymerpartikeln und dazwischengelagerten Arzneimittelpartikeln zu bilden; und dann
(b) Kombinieren der Partikelmischung mit einem überkritischen Gaslösungsmittel bei einem Druck, der ausreicht, um die Viskosität des Polymermaterials zu verringern, die Polymerpartikel miteinander zu verschmelzen und die Arzneimittelpartikel dazwischen zu erfassen, und aus der partikelförmigen Mischung ein Arzneimittel/Polymer-Verbundmaterial zu bilden, wobei der Hilfsstoff ein Porogen ist, wobei das Verfahren des Weiteren den Schritt des Inkontaktbringes des Verbundmaterials mit einem Lösungsmittel umfasst, um das Porogen zumindest teilweise zu solubilisieren und Poren in dem Verbundmaterial zu bilden.

2. Verfahren nach Anspruch 1, wobei der Kombinierschritt (b) in einer Form durchgeführt wird, so dass dadurch ein geformter Gegenstand aus dem Arzneimittel/Polymer-Verbundmaterial hergestellt wird.

3. Verfahren nach Anspruch 2, wobei der geformte Gegenstand ein Stent, ein Arzneimitteldepot oder ein biomedizinisches Implantat ist

4. Verfahren nach Anspruch 1, das des Weiteren den Schritt umfasst:
(c) Bilden eines geformten Gegenstandes aus dem Arzneimittel/Polymer-Verbundmaterial.

5. Verfahren nach Anspruch 4, wobei der Kombinierschritt (b) in einem Extruder durchgeführt wird

6. Verfahren nach Anspruch 4, wobei der Formschritt (c) durch Beschichten eines vorgeformten Substrats mit dem Arzneimittel/Polymer-Verbundmaterial durchgeführt wird.

7. Verfahren nach Anspruch 4, wobei der geformte Gegenstand ein Stent, ein Arzneimitteldepot oder ein biomedizinisches Implantat ist

8. Verfahren nach Anspruch 1, wobei das Arzneimittel ein Protein oder Peptid ist.

9. Verfahren nach Anspruch 1, das des Weiteren den Schritt des Beschichtens des Verbundmaterials mit einem Sekundärmaterial umfasst.

## Revendications

1. Procédé de formation d'un matériau composite médicament/polymère, comprenant les étapes de :
(a) mélange d'un matériau médicamenteux particulaire solide avec un matériau polymère particulaire solide, et un excipient pharmaceutique, pour former un mélange de particules de particules de polymère et de particules de médicament intercalées ; puis
(b) combinaison dudit mélange de particules avec un solvant gazeux supercritique à une pression suffisante pour réduire la viscosité dudit matériau polymère, fusionner lesdites particules de polymère les unes avec les autres et capturer lesdites particules de médicament entre elles et former un matériau composite médicament/polymère à partir dudit mélange particulaire, dans lequel ledit excipient est un agent porogène, ledit procédé comprenant en outre l'étape de mise en contact dudit matériau composite avec un solvant afin de solubiliser au moins partiellement ledit agent porogène et de former des pores dans ledit matériau composite.

2. Procédé selon la revendication 1, dans lequel ladite étape de combinaison (b) est réalisée dans un moule de sorte qu'un article façonné dudit matériau composite médicament/polymère soit ainsi produit.

3. Procédé selon la revendication 2, dans lequel ledit article façonné est un stent, un dépôt de médicament ou un implant biomédical.

4. Procédé selon la revendication 1, comprenant en outre l'étape de :
(c) formation d'un article façonné à partir dudit matériau composite médicament/polymère.

5. Procédé selon la revendication 4, dans lequel ladite étape de combinaison (b) est réalisée dans une extrudeuse.

6. Procédé selon la revendication 4, dans lequel ladite étape de formation (c) est réalisée en revêtant un substrat préformé avec ledit matériau composite médicament/polymère.

7. Procédé selon la revendication 4, dans lequel ledit article façonné est un stent, un dépôt de médicament ou un implant biomédical.

8. Procédé selon la revendication 1, dans lequel ledit médicament est une protéine ou un peptide.

9. Procédé selon la revendication 1, comprenant en outre l'étape de revêtement dudit matériau composite avec un matériau secondaire.
